# EUROPEAN PATENT SPECIFICATION

(11) **EP 2 394 645 B1**
(45) Date of publication and mention of the grant of the patent: **08.10.2014**
(21) Application number: 10738778.9
(22) Date of filing: 09.02.2010
(51) Int. Cl.: A61K 31/192, A61P 25/24, A61P 25/22, A61P 25/00, A61K 31/167, A61K 31/196

(54) **Medical use of 5-benzylamino salicylic acid derivatives or salts thereof**
Medizinische Verwendung von 5-Benzylamino Salicylsäurederivaten oder Salzen davon
Utilisation médicale de dérivés d'acide 5-benzylaminosalicylique ou de sels correspondants

(30) Priority: 09.02.2009 KR 20090010068; 08.02.2010 KR 20100011521
(43) Date of publication of application: 14.12.2011
(73) Proprietor: Neurotech Pharmaceuticals Co., Ltd., Suwon-si, Gyeonggi-do 443-270 (KR)
(72) Inventor: GWAG, Byoung-Joo, Suwon-si Gyeonggi-do 443-777 (KR); SON, Sun-Joo, Suwon-si Gyeonggi-do 441-822 (KR); NOH, Jae-Sung, Anyang-si Gyeonggi-do 431-070 (KR); SHIN, Jin-Hee, Seoul 137-772 (KR)
(74) Representative: Le Coupanec, Pascale A.M.P.
(86) International application number: PCT/KR2010/000783
(87) International publication number: WO 2010/090494

(56) References cited:
- WO-A1-01/79153
- WO-A1-86/03199
- WO-A1-94/13663
- WO-A1-2007/119973
- KR-B1- 910 002 945
- US-A1- 2006 258 721
- US-A1- 2007 298 129

## Description

### [TECHNICAL FIELD]

The present invention relates to the field of pharmaceutical composition for treating or preventing stress disorder or depression, comprising 5-benzylaminosalicylic acid derivative or its pharmaceutically acceptable salts.

### [BACKGROUND ART]

Stress occurs when a person can not respond appropriately to emotional or physical threats and is often accompanied by symptoms such as tissue damage, hemorrhage, infection, hypoglycemia, pain, etc. In addition, excessive stress is the cause of mental illness such as depression or anxiety disorders.

Depression, a stress-related mental disease, recurs frequently, tends to be chronic, and causes serious consequences such as suicide. Main symptoms of depression are depressed mood and emotion and appear along with resultant loss of sleep, appetite, and interest, and anxiety, helplessness, guilt, isolation, and futility, impulsivity towards suicide. Change in weight is severe and the behavior becomes very dull and slow. And, depression is accompanied by feelings of worthlessness, inappropriate guilt, and decreased concentration and memory. The depression patients feel chronically tired and can not sleep in many cases and have a bad night's sleep. In addition to changes in emotions, thoughts, and desires, physical symptoms such as headache, indigestion, stiff neck and shoulders, chest tightness and so on appear. In case of severe depression, delusions or hallucinations might occur.

It is known that depression is caused by not only by social and environmental factors such as several psychological factors, shock or stress etc. but also by biological factors such as disorders of nervous system or endocrine system.

Drug intakes such as antihypertensive drugs, anxiolytics, psychotropic drugs, and central nervous system stimulants can be a cause of depression. Diabetes, pancreatic cancer, or endocrine disease also can be a cause of depression.

Since the 1950s, tricyclic antidepressants (TCAs) began to be used as an effective first-line therapy for depression through the mechanism increasing concentration of serotonin and epinephrine in the synapse. Although the efficacy of TCAs is very good, central nervous system side effects such as sedation, confusion, delusions, compulsive disorder, headaches, sleep disturbances and cardiovascular side effects such as postural hypotension and tachycardia, and anticholinergic side-effects remain to be considerable problems. After that, since 1980s, launching of selective serotonin reuptake inhibitors (SSRIs) which have similar effects but superior in terms of side effects and safety compared with TCAs has brought great changes in the therapy of depression. Currently, fluoxetine, citalopram, sertraline, paroxetine, and escitalopram are commercially available, occupying about 70% of antidepressants.

However, these SSRIs also show headache, nausea, appetite inhibition, sexual dysfunction, sleep disturbances, fatigue, weight change, suicidal ideation and extrapyramidal side effects. Recently, antidepressant drugs with compound pharmacological actions have been used to enhance therapeutic effects including serotonin and norepinephrine reuptake inhibitors (SNRIs) such as venlafaxine and milnacipran, a noradrenergic and a specific serotonergic antidepressant (NSSA), mirtazapine, and norepinephrine and dopamine reuptake inhibitors (NDRIs), bupropion.

Clinical symptoms and medications of major depression tend to overlap with anxiety disorders including panic-agoraphobia syndrome, severe phobias, generalized anxiety disorder (GAD), social anxiety disorder, post-traumatic stress disorder (PTSD), and obsessive-compulsive disorder (OCD). In fact, antidepressants such as tricyclic antidepressants and serotonin reuptake inhibitors are used in the treatment for panic disorder, agoraphobia, obsessive-compulsive and traumatic stress disorder.

It is known by the document US 2007/0298199, the use of 2-hydroxy-alkylamino-benzoic acid derivatives for treating neurological diseases or ocular diseases.

### [DISCLOSURE]

### [TECHNICAL PROBLEM]

Accordingly, the object of the present invention is to provide compounds useful for treating or preventing stress disorder or depression.

### [TECHNICAL SOLUTION]

To achieve the object, the present invention provides 5-benzylaminosalicylic acid derivative selected from the group consisting of 2-hydroxy-5-[2-(4-trifluoromethyl-phenyl)-ethylamino]-benzoic acid, 2-acetoxy-5-[2-(4-trifluoromethyl-phenyl)-ethylamino]-benzoic acid or their pharmaceutically acceptable salts for use in the treatment or prevention of acute stress disorder, post-traumatic stress disorder or depression.

The present application also describes a pharmaceutical composition for treating or preventing stress disorder, depression or anxiety disorder, comprising 5-benzylaminosalicylic acid derivatives represented by the below chemical formula 1 or its pharmaceutically acceptable salts as effective agents: 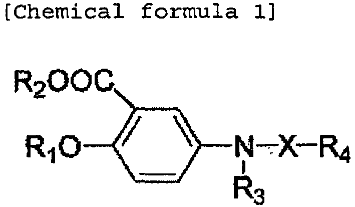 wherein,
X is CO, SO₂ or (CH₂)ₙ (where n is an integer of 1 to 5, inclusive);
R₁ is hydrogen, alkyl or alkanoyl;
R₂ is hydrogen or alkyl;
R₃ is hydrogen or an acetyl group; and
R₄ is phenyl group which is unsubstituted or substituted with one or more of the group consisting of nitro, halogen, haloalkyl, and C₁- C₅ alkoxy; or a pharmaceutically-acceptable salt thereof.

The present inventors have prepared and evaluated a lot of compounds, and succeeded in disclosing the fact that the 5-benzylaminosalicylic acid derivatives or its pharmaceutically acceptable salts are much useful for treating or preventing stress disorder, depression or anxiety disorder as well as safe.

In the present description, above 5-benzylaminosalicylic acid derivatives include 5-benzylaminosalicylic acid itself.

In the chemical formula 1, alkyl (including 'alkyl' of haloalkyl) may be C₁-C₅ alkyl, and more particularly C₁-C₃ alkyl. More specifically, alkyl may include methyl, ethyl, propyl, isopropyl, butyl, *sec*-butyl and tert-butyl. Alkoxy (including 'alkoxy' of haloalkoxy) may be C₁-C₅ alkoxy, and more particularly C₁-C₃ alkoxy. More specifically, alkoxy may include methoxy, ethoxy, and propanoxy. Halogen may include fluoride, chloride, bromide, and iodide. Alkanoyl may be C₂-C₁₀ alkanoyl, and more particularly C₃-C₅ alkanoyl. More specifically, alkanoyl may include ethanoyl, propanoyl, and cyclohexanecarbonyl.

Examples of the 5-benzylaminosalicylic acid derivative may include 2-hydroxy-5-phenethylamino-benzoic acid (compound 1), 2-hydroxy-5-[2-(4-trifluoromethyl-phenyl)-ethylamino]-benzoic acid (compound 2), 2-hydroxy-5-[2-(3-trifluoromethyl-phenyl)-ethylamino]-benzoic acid (compound 3), 5-[2-(3,5-bis-trifluoromethyl-phenyl)-ethylamino]-2-hydroxy-benzoic acid (compound 4), 2-hydroxy-5-[2-(2-nitro-phenyl)-ethylamino]-benzoic acid (compound 5), 5-[2-(4-chloro-phenyl)-ethylamino]-2-hydroxy-benzoic acid (compound 6), 5-[2-(3,4-difluoro-phenyl)-ethylamino]-2-hydroxy-benzoic acid (compound 7), 5-[2-(3,4-dichloro-phenyl)-ethylamino]-2-hydroxy-benzoic acid (compound 8), 5-[2-(4-fluoro-2-trifluoromethyl-phenyl)-ethylamino]-2-hydroxy-benzoic acid (compound 9), 5-[2-(2-fluoro-4-trifluoromethyl-phenyl)-ethylamino]-2-hydroxy-benzoic acid (compound 10), 2-hydroxy-5-[2-(4-methoxy-phenyl)-ethylamino]-benzoic acid (compound 11), 2-hydroxy-5-(2-o-tolyl-ethylamino)-benzoic acid (compound 12), 2-hydroxy-5-(3-phenyl-propylamino)-benzoic acid (compound 13), 2-hydroxy-5-[3-(4-trifluoromethyl-phenyl)-propylamino]-benzoic acid (compound 14), 5-[3-(4-fluoro-phenyl)-propylamino]-2-hydroxy-benzoic acid (compound 15), 5-[3-(3,4-dichloro-phenyl)-propylamino]-2-hydroxy-benzoic acid (compound 16), 2-hydroxy-5-(3-p-tolyl-propylamino)-benzoic acid (compound 17), 2-acetoxy-5-[2-(4-trifluoromethyl-phenyl)-ethylamino]-benzoic acid (compound 18), and 5-[2-(2-chloro-phenyl)-ethylamino]-2-hydroxy-benzoic acid (compound 19), 5-benzylaminosalicylic acid (compound 20), 5-(4-nitrobenzyl)aminosalicylic acid (compound 21), (5-(4-chlorobenzyl)aminosalicylic acid (compound 22), (5-(4-trifluoromethylbenzyl)aminosalicylic acid (compound 23), (5-(4-fluorobenzyl) aminosalicylic acid (compound 24), 5-(4-methoxybenzyl)aminosalicylic acid (compound 25), 5-(4-pentafluorobenzyl) aminosalicylic acid (compound 26), 5- (4-nitrobenzyl)amino-2-hydroxy ethylbenzoate (compound 27), 5-(4-nitrobenzyl)-N-acetylamino-2-hydroxy ethylbenzoate (compound 28), 5-(4-nitrobenzyl)-N-acetylamino-2-acetoxy ethylbenzoate (compound 29), 5-(4-nitrobenzoyl)aminosalicylic acid (compound 30), 5-(4-nitrobenzenesulfonyl)aminosalicylic acid (compound 31), 5-[2-(4-nitrophenyl)-ethyl]aminosalicylic acid, and (compound 32) 5-[3-(4-nitrophenyl)-n-propyl]aminosalicylic acid (compound 33).

The 5-benzylaminosalicylic acid derivative or its pharmaceutically acceptable salt of the present invention can be prepared by the reaction schemes represented in US 6,573,402.

The term "pharmaceutically acceptable salts" of the present invention means salts produced by non-toxic or little toxic acid or base. In case that the compound of the present invention is acidic, base addition salts of the compound of the present invention can be made by reacting the free base of the compound with enough amount of desirable base and adequate inert solvent. Pharmaceutically acceptable base addition salt includes sodium, potassium, calcium, ammonium, magnesium or salt made by organic amino. In case that the compound of the present invention is basic, acid addition salts of the compound of the compound can be made by reacting the free base of the compound with enough amount of desirable acid and adequate inert solvent. Pharmaceutically acceptable acid addition salt includes propionic acid, isobutylic acid, oxalic acid, malic acid, malonic acid, benzoic acid, succinic acid, suberic acid, fumaric acid, mandelic acid, phthalic acid, benzenesulfonic acid, p-tolylsulfonic acid, citric acid, tartaric acid, methanesulfonic acid, hydrochloric acid, bromic acid, nitric acid, carbonic acid, monohydrogencarbonic acid, phosphoric acid, monohydrogen-phosphoric acid, dihydrogen-phosphoric acid, sulfuric acid, monohydrogen-sulfuric acid, hydrogen iodide, and phosphorous acid. In addition, the pharmaceutically acceptable salt of the present invention includes a salt of amino acid like arginate and an analog of organic acid like glucuronic or galactunoric.

For example, a pharmaceutically acceptable salt of 2-hydroxy-5-[2-(4-trifluoromethyl-phenyl)-ethylamino]-benzoic acid (compound 2), one preferable example of the present invention, can be prepared by the below reaction scheme 1. However, the following reaction methods are offered by way of illustration.

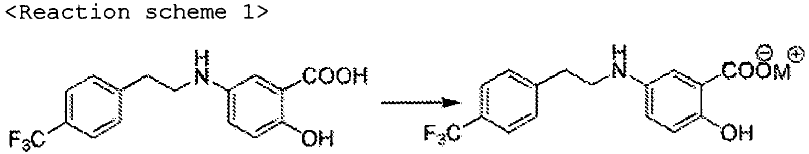

In the scheme, M is a pharmaceutically acceptable metal or basic organic compound such as diethylamine, lithium, sodium and potassium.

In more detail, diethylamine salt can be prepared by dissolving a compound in alcohol, adding dropwise diethylamine, stirring the mixture, distilling in vacuo, and crystallizing the residue by adding ether. Alkali metal salt can be made by preparing desirable salt with inorganic reagent like lithium hydroxide, sodium hydroxide, potassium hydroxide in solvent like alcohol, acetone, acetonitrile and then freeze-drying. In addition, according to the similar method, lithium salt can be made with lithium acetate, sodium salt can be made with sodium 2-ethylhexanoate or sodium acetate, and potassium salt can be made with potassium acetate.

Some of the compounds of the present invention may be hydrated form, and may exist as solvated or unsolvated form. A part of compounds according to the present invention exist as crystal form or amorphous form, and any physical form is included in the scope of the present invention. In addition, some compounds of the present disclosure may contain one or more asymmetric carbon atoms or double bond, and therefore exists in two or more stereoisomeric forms like racemate, enantiomer, diastereomer, geometric isomer. The present disclosure includes these individual stereoisomers of the compounds of the present disclosure.

The present application also describes a pharmaceutical composition comprising the 5-benzylaminosalicylic acid derivative represented by the above chemical formula 1 or its pharmaceutically acceptable salts; and pharmaceutically acceptable excipient or additive. The 5-benzylaminosalicylic acid derivative represented by the above chemical formula 1 or its pharmaceutically acceptable salts may be administered alone or with any convenient carrier, diluent, and a formulation for administration may be single-dose unit or multiple-dose unit.

The pharmaceutical composition may be formulated in a solid or liquid form. The solid formulation includes a powder, a granule, a tablet, a capsule, a suppository. Also, the solid formulation may further include, a diluent, a flavoring agent, a binder, a preservative, a disintegrating agent, a lubricant, a filler. The liquid formulation includes a solution such as water solution and propylene glycol solution, a suspension, an emulsion, and may be prepared by adding suitable additives such as a coloring agent, a flavoring agent, a stabilizer, a thickener, etc.

For example, a powder can be made by simply mixing the 5-benzylaminosalicylic acid derivative of the present invention and pharmaceutically acceptable excipients like lactose, starch, microcrystalline cellulose. A granule can be prepared as follows: mixing the compound or its pharmaceutically acceptable salt, a pharmaceutically acceptable diluent and a pharmaceutically acceptable binder such as polyvinylpyrrolidone, hydroxypropylcellulose; and wet-granulating with adequate solvent like water, ethanol, isopropanol, or direct-compressing with compressing power. In addition, a tablet can be made by mixing the granule with a pharmaceutically acceptable lubricant such as magnesium stearate, and tabletting the mixture.

The pharmaceutical composition may be administered in forms of oral formulation, injectable formulation (for example, intramuscular, intraperitoneal, intravenous, infusion, subcutaneous, implant), inhalable, intranasal, vaginal, rectal, sublingual, transdermal, topical depending on the disorders to be treated and the patient's conditions. The composition may be formulated in a suitable dosage unit comprising a pharmaceutically acceptable and non-toxic carrier, additive and/or vehicle, which all are generally used in the art, depending on the routes to be administered. Depot type of formulation being able to continuously release drug for desirable time also is described in the present application.

The present application also describes a use of the 5-benzylaminosalicylic acid derivative or its pharmaceutically acceptable salts for treating or preventing stress disorder, depression, or anxiety disorder. That is, the present application describes a pharmaceutical composition for treating or preventing stress disorder, depression, or anxiety disorder, comprising the 5-benzylaminosalicylic acid derivative represented by the above chemical formula 1 or its pharmaceutically acceptable salts. More specifically, the 5-benzylaminosalicylic acid derivative or its pharmaceutically acceptable salts may be used for treating or preventing stress disorder such as acute stress disorder, post-traumatic stress disorder, anxiety disorder such as social anxiety disorder, generalized anxiety disorder, panic disorder, agoraphobia, substance-induced anxiety disorder, anxiety disorder due to a general medical condition, or depression.

For use in treating stress disorder, depression, or anxiety disorder, the compound may be administered daily at a dose of approximately 0.01 mg/kg to approximately 100 g/kg, preferably approximately 0.1 mg/kg to approximately 10 g/kg. However, the dosage may be varied according to the patient's conditions (age, sex, body weight), the severity of patients in need thereof, the used effective components, diets. The compound of the present invention may be administered once a day or several times a day in divided doses, if necessary.

### [BRIEF DESCRIPTION OF THE DRAWINGS]

Figure 1 is the result evaluating the antidepressant effect of compound 2 using tail suspension test.
   *, p<0.05 vs vehicle group, BID (twice a day)
Figure 2 is the result evaluating the anti-stress effect of compound 2 using restraint stress model.
Figure 3 is the result showing the effect of the administration to compound 2 in open field test.
   *, p<0.05 vs vehicle group
Figure 4 is the result evaluating the antidepressant effect of compound 2 using forced swimming test.
   *, p<0.05 vs vehicle group
Figure 5 is the result evaluating the antidepressant effect of compound 2 using restraint stress test.
   * p<0.05 vs non-stressed vehicle (control 1) group
   # p<0.05 vs stressed vehicle (control 2) group
Figure 6 is the result evaluating the anxiolytic effect of compound 2 using stress-induced anxiety.
   * p<0.05 vs non-stressed vehicle (control 1) group
   # p<0.05 vs stressed vehicle (control 2) group

### [EXAMPLES]

Hereinafter, the present invention is described in considerable detail to help those skilled in the art understand the present invention. The following examples are offered by way of illustration.

### <Example 1> Evaluation of antidepressant effect of compound 2 on tail suspension test (TST)

The effectiveness of the compound was evaluated using a tail-suspension test as in vivo method for depression. In this test immobility time of animals typically for 5-10 minutes is recorded using a manual or an automated device. The mouse suspended by the tail shows alternate activity (movement) and immobility and drugs with antidepressant action commonly reduce the immobility time in this test.

Male ICR mice weighing 28-30g of body weight were used throughout the study. Experimental animals were kept in an animal breeding room with a 12 h dark/12 h light cycle and accommodated by 8 mice per cage supplied freely with water and feeding. The mice consisted of 8 animals per group were randomly allocated to the treatment groups.

Compound 2 was suspended in 10 % lutrol solution and the all suspended solutions were administered orally to the mice twice daily at a volume of 4 ml/kg. The control group was treated only with 10 % lutrol solution in the same manner. All the treatment of drugs or vehicle to the animals were given 1 hour before the test (n=8/group).
1. Control 1 (vehicle, 10% lutrol)
2. Compound 2 1.25 mg/kg (oral administration, twice daily)
3. Compound 2 2.5 mg/kg (oral administration, twice daily)
4. Compound 2 5 mg/kg (oral administration, twice daily)
5. Compound 2 12.5 mg/kg (oral administration, twice daily)
6. Compound 2 25 mg/kg (oral administration, twice daily)
7. Compound 2 50 mg/kg (oral administration, twice daily)

This test was evaluated according to the method described in a previous publication [Psycopharmacology 85, 367-370, Steru et al. (1985)]. At the day of experiment, the mice were acclimatized in the testing room over 60 minutes and given with the compound, and then the test was conducted. After 1 h after drug treatment, the mice were taped with experimental adhesive tape, at approximately 1 cm from the tip of the tail. The mice were individually suspended on an experimental shelf about 58 cm above a table top by attaching the adhesive tape at the tip of the tail to the experimental shelf. And after that, total immobility time was measured for 6 min in the absence of noise. The total immobility time was measured during the final 4 min of a 6 min test session and the data were expressed as mean and analyzed by one-way ANOVA followed by Tukey's HSD (Honestly Significant Difference) post hoc comparison.

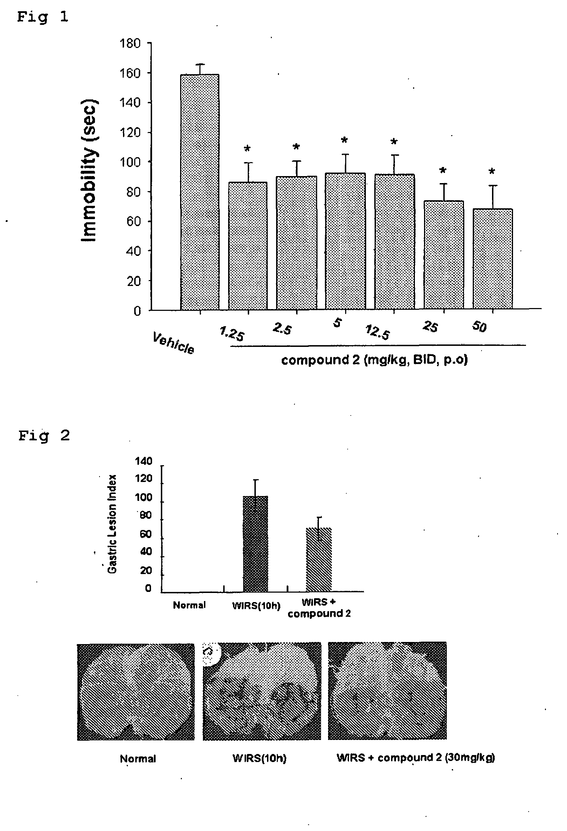

As shown in figure 1, all the groups treated with compound 2 decreased immobility time during experimental time of 4 minutes. In groups treated with compound 2 at doses of 1.25 mg/kg, 2.5 mg/kg, 5 mg/kg, 12.5 mg/kg, 25 mg/kg, and 50 mg/kg, the total immobility time were significantly decreased by 46%, 43%, 42%, 38%, 54%, and 57%, respectively, compared with the control group. Thus, it can be known that compound 2 according to the present invention has antidepressant activity.

### <Example 2> the effect of compound 2 in stress models

### 2-1. Water immersion restraint stress (WIRS) induction

The Sprague-Dawley (SD) rats weighing 200g of body weight were deprived of food for over 24 hours and then were given with the experimental compound. After 1 hour, the rats were placed in a stress cage and immersed into water at 24°C for 10 hours to induce WIRS

### 2-2. the effect of compound 2 on gastric damage in stress model

The SD rats weighing 200g of body weight were deprived of food for 24 h and then were given with 30 mg/kg of compound 2. After 1 hour, the rats were placed in a stress cage and immersed into water for 10 hours. After WIRS for 10 hours, the rats were sacrificed under anesthesia with ether. Each stomach was isolated, soaked and fixed in 10 ml of 2% formalin solution for 10 minutes, and then opened along the greater curvature of the stomach. After spreading the stomach, stress-induced hemorrhagic lesions were examined macroscopically, and the results were shown in figure 2.

As shown in figure 2, treatment with 30 mg/kg of compound 2 definitely reduced WIRS-induced gastric lesion index.

### <Example 3> The effect of compound 2 on locomotor activity using open field test

The locomotor activity using open field test measured to know influence of drug treatment on activity in animals. Male ICR mice weighing 20-23g of body weight were used throughout the study.

Experimental animals were kept in an animal breeding room with a 12 h dark/12 h light cycle and accommodated by 8 mice per cage supplied freely with water and feeding. The mice consisted of 8 animals per group were randomly allocated to the treatment groups.

Compound 2 was suspended in 10 % lutrol solution and all the suspended solutions were administered orally to the mice twice daily at a volume of 5 ml/kg. The control group was treated only with 10 % lutrol solution in the same manner. All the treatment of drugs or vehicle to the animals were given 1 hour before the test (n=8/group).
1. Control 1 (vehicle, 10% lutrol)
2. Compound 2 1.25 mg/kg (oral administration, twice daily)
3. Compound 2 2.5 mg/kg (oral administration, twice daily)
4. Compound 2 5 mg/kg (oral administration, twice daily)
5. Compound 2 12.5 mg/kg (oral administration, twice daily)
6. Compound 2 25 mg/kg (oral administration, twice daily)
7. Compound 2 50 mg/kg (oral administration, twice daily)

The open field was consisted of square arena (58 cm x 58 cm) surrounded by a 30 cm height wall. The floor of the arena was divided into 12 equal squares. At the start of each trial, a mouse was placed in a corner of the field and was allowed to freely move the arena and the moving trajectory for 5 min was measured. The behavior of animals was recorded and analyzed using a videotracking software (Etho Vision 3.0, Noldus Information Technology, Leesburg, VA) attached to the ceiling above the middle of the arena.

The collected data were analyzed by one-way ANOVA followed by Tukey's HSD (Honestly Significant Difference) *post hoc* comparison.

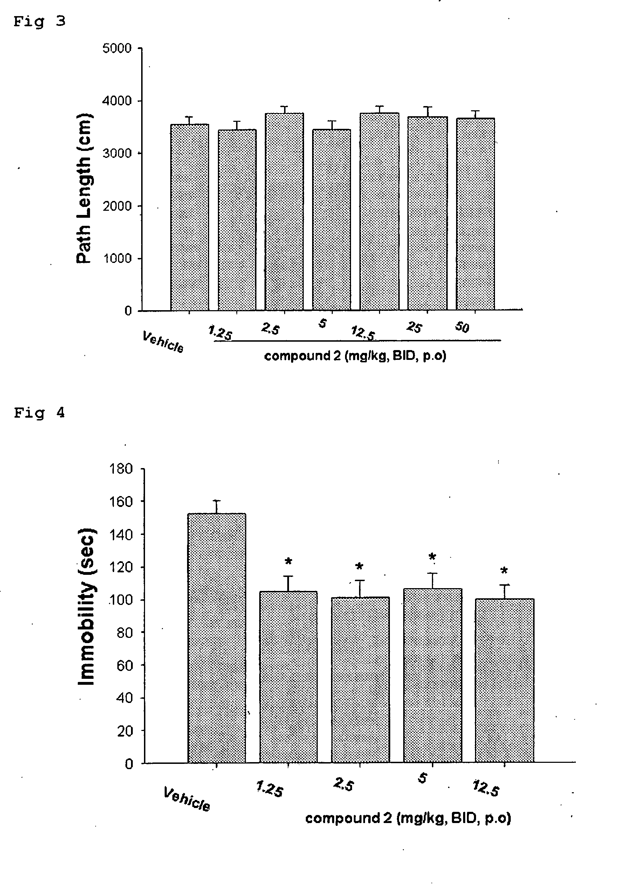

As shown in figure 3, locomotor activity was not different among groups, suggesting that compound 2 shows antidepressant activity without influencing on the locomotor activity.

### <Example 4> Evaluation of antidepressant effect on forced swimming test

Forced swimming test is used to select compounds with therapeutic efficacy for depression. An animal located in a cylinder filled with water shows various avoidance behaviors or immobility. The immobility time is counted whenever the animal remained floating passively in the water without struggling except minimal movement to protrude its head above the water surface. Antidepressants significantly improve the avoidance behavior or immobility.

Nine-week-old male C57BL/6 mice weighing 20-23g of body weight were used through this study. Experimental animals were kept in an animal breeding room with a 12 h dark/12 h light cycle and accommodated by 8 mice per cage supplied freely with water and feeding. The mice consisted of 8 animals per group were randomly allocated to the treatment groups.

Compound 2 was suspended in 10 % lutrol solution and all the suspended solutions were administered orally to the mice twice daily at a volume of 5 ml/kg. The control group was treated only with 10 % lutrol solution in the same manner. All the treatment of drugs or vehicle to the animals were given 1 hour before the test (n=8/group).
1. Control 1 (vehicle, 10% lutrol)
2. Compound 2 1.25 mg/kg (oral administration, twice daily(BID))
3. Compound 2 2.5 mg/kg (oral administration, twice daily(BID))
4. Compound 2 5 mg/kg (oral administration, twice daily(BID))
5. Compound 2 12.5 mg/kg (oral administration, twice daily(BID))

At the day of the experiment, the mice were acclimatized in the testing room over 60 minutes, orally given with the compound 2 or vehicle control, and received the forced swimming test. For the forced swimming test, a mouse was individually forced to swim inside an open glass cylindrical bath with height of 26 cm and diameter of 14 cm filled with water at 24±1 °C up to 16 cm high from the bottom so that the mouse tail could not reach the bottom. One hour after the mouse were orally given compound 2 and control vehicle, then the mice were exposed to forced swimming for 6 min in the water bath and their movement was recorded using a video camera system. The total duration of immobility was measured as a mean during the final 4 min of a 6-min test session and the data were statistically analyzed with one-way ANOVA followed by Tukey's HSD (Honestly Significant Difference) post hoc comparison.

The results were represented in figure 4. All the groups treated with compound 2 decreased immobility time during the test time of 4 minutes. Immobility time in the groups treated with compound 2 was significantly decreased compared with the vehicle control group, showing 31%, 34%, 30%, and 34% at doses of 1.25 mg/kg, 2.5 mg/kg, 5 mg/kg, and 12.5 mg/kg, respectively.

### <Example 5> Evaluation of antidepressant effect on repeated restraint stress test

Currently, two aspects of depression, efficacy of antidepressant and response on stress, are mainly utilized for the depression studies using experimental animals.

In case of a realistic animal model of depression, in which symptoms corresponding to depression are created by exposing animals to a similar situation to be able to evoke corresponding human diseases, a study on the physiological defects becomes possible, which reside as causes in the diseases. Such realistic animal models of depression evoke depression to animals mainly by adding stress such as isolation, electric shock, forced swimming, environmental changes, and temperature changes and so on.

To test the efficacy of compounds on a stress-induced depression a restraint stress animal model was used. After evoking depression symptoms in a mouse corresponding to a human depression by exposing a stressed condition that it was placed in a narrow space to be unable to move, whether the compounds have any anti-depression effects were tested after treatment to this animal.

Nine-week-old male C57BL/6 mice weighing 20-23g of body weight were used through this study. Experimental animals were kept in an animal breeding room with a 12 h dark/12 h light cycle and accommodated by 5~6 mice per cage supplied freely with water and feeding. The mice consisted of 11 animals per group were randomly allocated to the treatment groups.

Compound 2 was suspended in 10 % lutrol solution and all the suspended solutions were administered orally to the mice twice daily at a volume of 5 ml/kg. The control group was treated only with 10 % lutrol solution in the same manner. All the treatment of drugs or vehicle to the animals were given 1 hour before the test (n=11/group).
1. Control 1 (vehicle, 10% lutrol)
2. Compound 2 2.5 mg/kg (oral administration, twice daily(BID))
3. Compound 2 5 mg/kg (oral administration, twice daily(BID))
4. Control 2 (10% lutrol) + restraint stress
5. Compound 2 2.5 mg/kg (oral administration, twice daily(BID))+ restraint stress
6. Compound 2 5 mg/kg (oral administration, twice daily(BID))+ restraint stress

Animals were subjected to restraint-stress one hour after compound 2 or 10% lutrol was administered orally. Mice were exposed to restraint stress everyday 2 hr for 14 days and received a forced swim test such as <Example 4> as an index for depression.

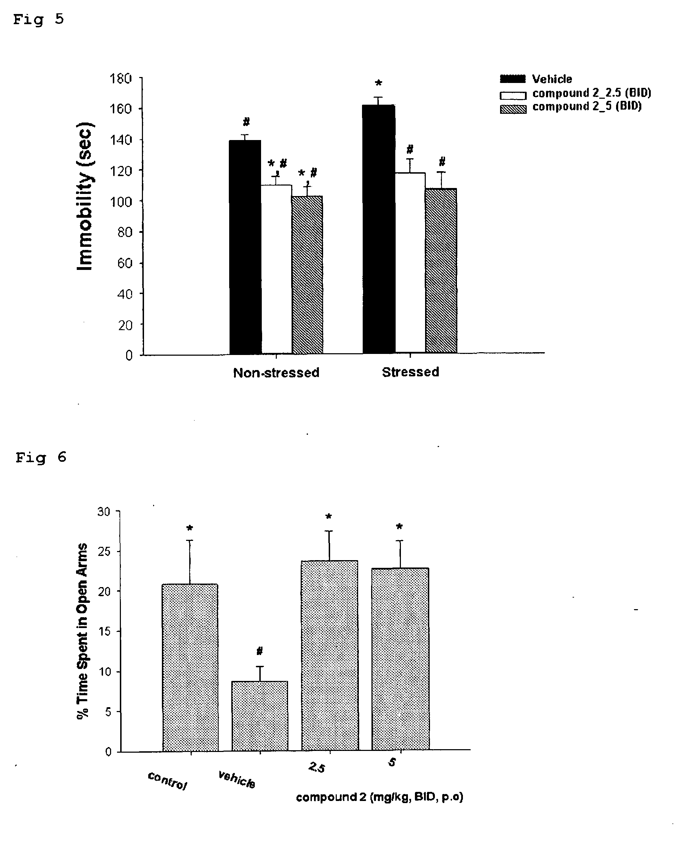

The results were shown in figure 5. The immobility time was significantly increased by 16% in the stressed control group which received stress for 2 weeks (control 2) compared with unstressed control group (control 1) However, immobility time in all the groups treated with compound 2 was significantly decreased at doses of 2.5 mg/kg and 5 mg/kg by 32% and 39%, respectively, compared with stressed control group (control 2)

In addition, to determine the effect of long-term treatment of compound 2 on depression, a forced swimming test was performed after administration of compound 2 for 2 weeks. Immobility time was significanly decreased in groups treated with compound 2 at doses of 2.5 mg/kg and 5 mg/kg by 21% and 26%, respectively, compared with unstressed group (control 1).

### <Example 6> Anxiolytic effect of compound 2 on stress-induced anxiety

To evaluate the effect of compound 2 on stress-induced anxiety, we used a restraint stress animal model. Anxiety was evoked by exposing stress that a mice was restrained to be immovable in a narrow space, and whether the compound could show anxiolytic effect after administration the animals.

When experimental animals are exposed to stress, various physiological changes occur, which can be measured through several behavioral tests. In this study, anxiety of experimental animals was evaluated by an elevated plus maze (EPM). EPM apparatus consisted of two open arms, and two closed arms which are horizontally crossed. It is designed that the cross path is located 60 cm above the floor for the animals to feel anxiety.

Nine-week-old male C57BL/6 mice weighing 20-23g of body weight were used through this study. Experimental animals were kept in an animal breeding room with a 12 h dark/12 h light cycle and accommodated by 8 mice per cage supplied freely with water and feeding. The mice consisted of 16 animals per group were randomly allocated to the treatment groups.

Compound 2 was suspended in 10 % lutrol solution and all the suspended solutions were administered orally to the mice at a volume of 5 ml/kg. The control group was treated only with 10 % lutrol solution in the same manner. All the treatment of drugs or vehicle to the animals were given 1 hour before the test (n=16/group).
1. Control 1 (10% lutrol)
2. Control 2 (10% lutrol) + restraint stress
3. Compound 2 2.5 mg/kg (oral administration, twice daily(BID))+ restraint stress
4. Compound 2 5 mg/kg (oral administration, twice daily(BID))+ restraint stress

Animals were subjected to restraint-stress one hour after compound 2 or 10% lutrol was administered orally. Mice were exposed to restraint stress everyday 2 hr for 14 days and received an elevated plus maze test as an index for anxiety.

At the day of experiment, animals exposed to restraint stress after animals were acclimatized to testing room over 60 minutes, and then were orally administered with the compound. One hour after administration the oral administration of compound 2 or lutrol, experimental animals were placed at the center of EPM and the animal behaviors were recorded during total 5 minutes using a camera mounted on the ceiling and a computer program [etho vision 3.0 (Noldus Information Technology)]. The collected data were expressed as % time spent in open arms and the statistical analysis was done using one-way ANOVA followed by LSD (Least significant difference) post hoc comparisons.

The results were shown in figure 6. When % time spent in open arm was observed, the rate showed 21% and 9% in unstressed (control 1) and stressed (control 2) groups, respectively, suggesting that anxiety was induced by current chronic stress. On the other hand, % time spent in open arm was significantly increased in the group treated with compound 2 at doses of 2.5 mg/kg and 5 mg/kg by 24% and 23%, respectively, compared with the stressed group (control 2), showing similar levels to the unstressed group (control 1).

### [INDUSTRIAL APPLICABILITY]

The present invention provides 5-benzylaminosalicylic acid derivative selected from the group consisting of 2-hydroxy-5-[2-(4-trifluoromethyl-phenyl)-ethylamino]-benzoic acid, 2-acetoxy-5-[2-(4-trifluoromethyl-phenyl)-ethylamino]-benzoic acid or their pharmaceutically acceptable salts for use in the treatment or prevention of acute stress disorder, post-traumatic stress disorder or depression.

## Claims

1. 5-benzylaminosalicylic acid derivative selected from the group consisting of 2-hydroxy-5-[2-(4-trifluoromethyl-phenyl)-ethylamino]-benzoic acid, 2-acetoxy-5-[2-(4-trifluoromethyl-phenyl)-ethylamino]-benzoic acid or their pharmaceutically acceptable salts for use in the treatment or prevention of acute stress disorder, post-traumatic stress disorder or depression.

## Patentansprüche

1. 5-Benzylaminosalicylsäurederivat, das aus der Gruppe ausgewählt wird, bestehend aus 2-Hydroxy-5-[2-(4-Trifluor-methyl-Phenyl)-Ethylamino]-Benzoesäure, 2-Acetoxy-5-[2-(4-Trifluormethyl-Phenyl)-Ethylamino]-Benzoesäure und deren pharmazeutisch annehmbaren Salze zur Verwendung in der Behandlung oder Prävention einer akuten Belastungsstörung, einer posttraumatischen Belastungsstörung oder einer Depression.

## Revendications

1. Dérivé d'acide 5-benzylaminosalicylique choisi dans l'ensemble constitué par l'acide 2-hydroxy-5-[2-(4-trifluorométhylphényl)éthylamino]benzoïque, l'acide 2-acétoxy-5-[2-(4-trifluorométhylphényl)éthylamino]-benzoïque, et leurs sels pharmaceutiquement acceptables, pour utilisation dans le traitement ou la prévention d'un trouble de stress aigu, d'un trouble de stress post-traumatique, ou d'une dépression.
